# EUROPEAN PATENT APPLICATION

(11) **EP 4 606 274 A1**
(43) Date of publication of application: **27.08.2025**
(21) Application number: 23879594.2
(22) Date of filing: 03.10.2023
(51) Int. Cl.: A47J 27/00, G06Q 10/00, G16H 10/00, H04M 11/00

(54) **CONTROL SYSTEM, CONTROL METHOD, CONTROL PROGRAM, INFORMATION PRESENTATION SYSTEM, INFORMATION PRESENTATION METHOD, AND INFORMATION PRESENTATION PROGRAM**

(30) Priority: 21.10.2022 JP 2022169107
(71) Applicant: Panasonic Intellectual Property Management Co., Ltd., Kadoma-shi, Osaka 571-0057 (JP)
(72) Inventor: KONDO, Kenji, Kadoma-shi, Osaka 571-0057 (JP); TSUJI, Kiyotaka, Kadoma-shi, Osaka 571-0057 (JP); OKUMURA, Yasuaki, Kadoma-shi, Osaka 571-0057 (JP); YAMAJI, Satoru, Kadoma-shi, Osaka 571-0057 (JP)
(74) Representative: Grünecker Patent- und Rechtsanwälte PartG mbB
(86) International application number: PCT/JP2023/035994
(87) International publication number: WO 2024/084947

(57) **Abstract**

A control system (10) includes an acquisition unit (121), an information processing unit (122), and an output unit (123). The acquisition unit (121) acquires health condition information, and dish information indicating a dish related to the health condition information. The information processing unit (122) acquires cooking parameter information, including one or more cooking parameters, based on the acquired health condition information and dish information. The output unit (123) outputs a control signal, including the acquired cooking parameter information, to a cooking control unit (150), which controls a cooking device (100). The cooking parameter information includes a cooking parameter for altering a chemical component corresponding to the health condition information during a cooking process performed by the cooking device (100).

## Description

### Technical Field

The present disclosure relates to a control system and the like for controlling a cooking device.

### Background Art

PTL 1 discloses a method of providing meal menus. In this method, the computer performs a process of receiving a first keyword related to health and a process of acquiring information on a functional component related to the first keyword from a first database. In this method, the computer also performs a process of searching a second database for a meal menu using ingredients related to the acquired information on the functional component and a process of providing the retrieved meal menu to the user.

### Citation List

### Patent Literature

PTL 1: Japanese Patent Publication No. 5908359

### Summary of Invention

The present disclosure provides a control system and the like for facilitating cooking dishes containing chemical components related to a user's health-related preferences.

A control system according to one aspect of the present disclosure includes: an acquirer that acquires health condition information and dish information indicating a dish related to the health condition information; an information processor that acquires cooking parameter information, including one or more cooking parameters, based on the acquired health condition information and dish information; and an outputter that outputs a control signal, including the acquired cooking parameter information, to a cooking controller that controls a cooking device, wherein the cooking parameter information includes a cooking parameter for altering a chemical component corresponding to the health condition information during a cooking process performed by the cooking device.

This comprehensive or specific aspect may be implemented as a device, method, integrated circuit, computer program, or computer-readable recording medium, and may be implemented as any combination of a device, system, method, integrated circuit, computer program, and computer-readable recording medium. The computer-readable recording medium includes a non-volatile recording medium such as, for example, CD-ROM (Compact Disc-Read Only Memory).

According to the present disclosure, it is easy to cook a dish containing a chemical component related to a user's health-related preferences.

### Brief Description of Drawings

[Fig. 1] Fig. 1 is a block diagram illustrating an overall configuration including a control system and an information provision system according to an embodiment.
[Fig. 2] Fig. 2 is a diagram illustrating an example of first information stored in a first database.
[Fig. 3] Fig. 3 is a diagram illustrating an example of second information stored in a second database.
[Fig. 4] Fig. 4 is a diagram illustrating an example of third information stored in a third database.
[Fig. 5] Fig. 5 is a flowchart illustrating the overview of the operation of the control system according to the embodiment.
[Fig. 6] Fig. 6 is a sequence diagram illustrating the overview of the operation of each of the control system and the information provision system according to the embodiment.
[Fig. 7] Fig. 7 is a diagram illustrating a first example of a first image displayed on a display unit.
[Fig. 8] Fig. 8 includes diagrams each illustrating a second example of the first image displayed on the display unit.
[Fig. 9] Fig. 9 is a diagram illustrating a third example of the first image displayed on the display unit.
[Fig. 10] Fig. 10 includes diagrams each illustrating a fourth example of the first image displayed on the display unit.
[Fig. 11] Fig. 11 includes diagrams each illustrating a first example of a second image displayed on the display unit.
[Fig. 12] Fig. 12 is a diagram illustrating an example of an image displayed on the display unit.
[Fig. 13] Fig. 13 includes diagrams each illustrating a second example of the second image displayed on the display unit.
[Fig. 14] Fig. 14 is a diagram illustrating a first example of a third image displayed on the display unit.
[Fig. 15] Fig. 15 is a diagram illustrating a second example of the third image displayed on the display unit.
[Fig. 16] Fig. 16 includes diagrams each illustrating a third example of the third image displayed on the display unit.
[Fig. 17] Fig. 17 is a diagram illustrating an example of a fourth image displayed on the display unit.
[Fig. 18] Fig. 18 is a flowchart illustrating a process of generating a trained model.
[Fig. 19] Fig. 19 is a diagram illustrating an example of evaluation items for sensory evaluation.
[Fig. 20] Fig. 20 is a diagram illustrating an example of measured data of a chemical component.
[Fig. 21] Fig. 21 includes diagrams each illustrating an example of a trained model.

### Description of Embodiments

### (Underlying Knowledge Forming Basis of the Present Disclosure)

First, the inventors' point of view will be described below.

Conventionally, as disclosed in PTL 1, there is a known method of providing a user with a dish containing components (chemical components) that can have a positive effect on health, corresponding to the user's health-related preferences. This method, however, provides the user with a dish using ingredients containing the above-mentioned chemical components. For this reason, this method always requires the preparation of ingredients containing the above-mentioned chemical components in order for the user to cook a dish corresponding to the user's health-related preferences. That is, there is a problem that, when the above-mentioned ingredients are rare ingredients or special ingredients that are difficult to prepare, it is difficult for the user to cook the presented dish.

Additionally, the chemical components may alter due to heat or pressure. However, this method takes no account of changes in the above-mentioned chemical components caused by heat or pressure during cooking under heat or pressure.

In light of the above, the inventors have come to create the present disclosure.

A control system according to one aspect of the present disclosure includes: an acquirer that acquires health condition information and dish information indicating a dish related to the health condition information; an information processor that acquires cooking parameter information, including one or more cooking parameters, based on the acquired health condition information and dish information; and an outputter that outputs a control signal, including the acquired cooking parameter information, to a cooking controller that controls a cooking device, wherein the cooking parameter information includes a cooking parameter for altering a chemical component corresponding to the health condition information during a cooking process performed by the cooking device.

This makes it easier to cook a dish that contains a chemical component related to a user's health-related preferences.

Additionally, for example, the health condition information may include information indicating the chemical component corresponding to the health condition information.

This makes it easier to cook a dish that contains a chemical component related to a user's health-related preferences.

In addition, for example, the one or more cooking parameters may include either one or more first cooking parameters corresponding to a dish with a first sensory evaluation value, or one or more second cooking parameters corresponding to a dish with a second sensory evaluation value different from the first sensory evaluation value; and the one or more second cooking parameters may be one or more parameters in which a part or all of the one or more first cooking parameters have been modified.

This makes it easier to cook a dish that contains a chemical component related to a user's health-related preferences.

Additionally, for example, the dish information may indicate the dish in which ingredients are combined.

Furthermore, for example, the information processor may further acquire information indicating at least one of a type of an ingredient, a quantity of the ingredient, or a processed state of the ingredient.

This makes it easier to cook a dish that contains a chemical component related to a user's health-related preferences.

In addition, for example, the cooking parameter information may include a cooking parameter for increasing or decreasing a chemical component corresponding to the health condition information during a cooking process performed by the cooking device.

This makes it easier to cook a dish that contains a chemical component related to a user's health-related preferences.

Furthermore, for example, the chemical component may include at least one of a component that can have a positive effect on health or a component that can have a negative effect on health.

This makes it easier to cook a dish that contains a chemical component related to a user's health-related preferences.

Additionally, for example, the one or more cooking parameters may include at least one of a temperature parameter indicating a temperature of the cooking device, or a time parameter indicating a cooking time of the cooking device.

This makes it easier to cook a dish that contains a chemical component related to a user's health-related preferences.

Furthermore, for example, the one or more cooking parameters may include a pressure parameter indicating a pressure of the cooking device.

This makes it easier to cook a dish that contains a chemical component related to a user's health-related preferences.

In addition, for example, the information processor may acquire the cooking parameter information using a trained model, and the trained model may be machine-learned to take information indicating a dish and the chemical component as input, and to output the one or more cooking parameters.

This makes it easier to cook a dish that contains a chemical component related to a user's health-related preferences.

Furthermore, a control method according to one aspect of the present disclosure is a control method executed by a computer, including: acquiring health condition information; acquiring dish information indicating a dish related to the health condition information; acquiring cooking parameter information, including one or more cooking parameters, based on the acquired health condition information and dish information; and outputting a control signal including the acquired cooking parameter information, wherein the cooking parameter information includes a cooking parameter for altering a chemical component corresponding to the health condition information.

This makes it easier to cook a dish that contains a chemical component related to a user's health-related preferences.

Additionally, a control program according to one aspect of the present disclosure causes a computer to execute: acquiring health condition information; acquiring dish information indicating a dish related to the health condition information; acquiring cooking parameter information, including one or more cooking parameters, based on the acquired health condition information and dish information; and outputting a control signal including the acquired cooking parameter information, wherein the cooking parameter information includes a cooking parameter for altering a chemical component corresponding to the health condition information.

This makes it easier to cook a dish that contains a chemical component related to a user's health-related preferences.

In addition, an information provision system according to one aspect of the present disclosure includes: a display; and a display controller that causes the display to display a first image that receives input of health condition information, to display a second image that receives input of dish information indicating a dish related to the input health condition information, and to display a third image that represents recipe information based on the input health condition information and dish information, wherein the recipe information includes at least one of pre-process information related to a pre-process preceding a cooking process in a cooking device, or post-process information related to a post-process following the cooking process in the cooking device.

This makes it easier to cook a dish that contains a chemical component related to a user's health-related preferences.

Furthermore, for example, the display controller may further cause the display unit to display a fourth image that represents health advice information indicating health advice based on the input health condition information and dish information.

This makes it easier to cook a dish that contains a chemical component related to a user's health-related preferences.

Additionally, an information provision method according to one aspect of the present disclosure is an information provision method executed by a computer, including: acquiring health condition information; acquiring dish information indicating a dish related to the health condition information; and outputting recipe information based on the acquired health condition information and dish information, wherein the recipe information includes at least one of pre-process information related to a pre-process preceding a cooking process in a cooking device, or post-process information related to a post-process following the cooking process in the cooking device.

This makes it easier to cook a dish that contains a chemical component related to a user's health-related preferences.

In addition, an information provision program according to one aspect of the present disclosure causes a computer to execute: acquiring health condition information; acquiring dish information indicating a dish related to the health condition information; and outputting recipe information based on the acquired health condition information and dish information, wherein the recipe information includes at least one of pre-process information related to a pre-process preceding a cooking process in a cooking device, or post-process information related to a post-process following the cooking process in the cooking device.

This makes it easier to cook a dish that contains a chemical component related to a user's health-related preferences.

It can also be realized as a computer program that causes a computer to execute a characteristic process included in the control method of the present disclosure. Needless to say, such a computer program can be distributed via a computer-readable non-transitory recording medium such as a CD-ROM or through a communication network such as the Internet. The same applies to the information provision method of the present disclosure.

Hereinafter, an embodiment will be specifically described with reference to the drawings.

Note that the embodiment described hereinafter is all illustrative of comprehensive or specific examples of the present disclosure. The numerical values, shapes, materials, elements, the arrangement locations and connection forms of elements, steps, the order of steps, and the like discussed in the following embodiment are merely examples and are not intended to limit the present disclosure. Additionally, any elements in the following embodiment that are not described in the independent claims indicating the highest-level concept are described as optional elements. In addition, the contents of each of all embodiments can be combined. Furthermore, the drawings are schematic drawings and may not necessarily drawn to scale. In addition, the same reference numerals are used for the same component parts in each of the drawings.

Additionally, the control system according to the embodiment of the present disclosure may be configured such that all elements are included in a single computer, or it may be configured as a system in which elements are distributed across computers. The same applies to the information provision system according to the embodiment of the present disclosure.

### (Embodiment)

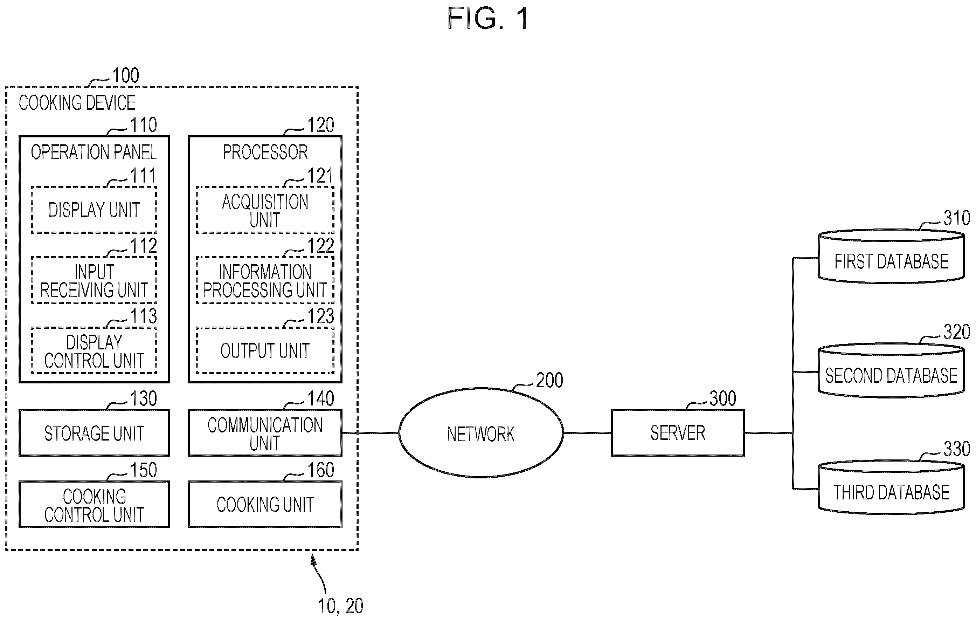

Hereinafter, a control system 10 (control method or control program) and an information provision system 20 (information provision method or information provision program), according to an embodiment of the present disclosure, will be described in detail using the drawings. Fig. 1 is a block diagram illustrating an overall configuration including the control system 10 and the information provision system 20 according to the embodiment.

The control system 10 and the information provision system 20 are both configured using a computer, such as a personal computer or a server. That is, both the control system 10 and the information provision system 20 may be realized, for example, by cloud computing. In the embodiment, the control system 10 and the information provision system 20 are described as being realized by a computer built in a cooking device 100. Additionally, in the embodiment, the cooking device 100 is described as being connected to a server 300 via a network 200, such as the Internet.

The cooking device 100 is, for example, a home appliance for cooking, such as a pressure cooker, or a commercial cooking device. In the embodiment, the cooking device 100 is a home appliance for cooking and is configured with a single enclosure. Note that, when the cooking device 100 is a commercial cooking device, the cooking device 100 may be configured with a single enclosure or may be configured with enclosures where functional components of the cooking device 100 are dispersed. In this case, the control system 10 and the information provision system 20 may be configured, for example, by a controller of the cooking device 100 or by an information terminal such as a smartphone.

The cooking device 100 falls under one of four types of devices: pressure cooker type, superheated steam type, IH (Induction Heating) cooking type, and container type.

The pressure cooker type device is a device that allows independent control of temperature and pressure within a sealed space, and is capable of cooking by boiling or stewing ingredients in a container. The pressure cooker type device can further perform cooking with water (described below) in its liquid state in a range that exceeds the upper limit of the temperature (e.g., 120 degrees Celsius) and the upper limit of the pressure (e.g., 0.2 MPa) utilized in commonly-used pressure cookers.

The superheated steam type device is a device that allows control of at least temperature, out of temperature and pressure, in a sealed or open space, and, by ejecting the generated superheated steam onto ingredients, it can perform cooking by steaming or grilling the ingredients. The superheated steam type device can further perform cooking that utilizes steam (described later) in its gas state in a range that exceeds the upper limit of the temperature (e.g., 120 degrees Celsius) and the upper limit of the pressure (e.g., 0.2 MPa) utilized in commonly-used pressure cookers. For the above-mentioned upper limits of temperature and pressure, see "http://www.apajapan.org/JPCC/pdf/poc01.pdf".

The IH cooking type device is a device that heats cookware, such as a frying pan, and is capable of cooking by grilling ingredients placed on the cookware. The container type device is a device configured to be capable of storing ingredients, with temperature control while the ingredients are stored, and is capable of fermenting or aging ingredients used when cooking items such as soy sauce or miso.

Here, cooking in a range that exceeds the upper limit (e.g., 120 degrees Celsius) of the temperature used in commonly-used pressure cookers has the advantage of facilitating hydrolysis because the ionic product is larger than cooking in the normal range (100 degrees Celsius). For example, in cooking within this range, the proteins contained in the ingredients are more easily hydrolyzed into peptides and amino acids. Additionally, for example, in cooking within this range, the collagen contained in the ingredients is more easily broken down into gelatin. For example, in cooking within this range using water in its liquid state, it becomes easier to extract bone marrow, including collagen, from bones such as beef bones, pork bones, or chicken carcasses.

The cooking device 100 includes an operation panel 110, a processor 120, a storage unit 130, a communication unit 140, a cooking control unit 150, and a cooking unit 160. Additionally, the operation panel 110 includes a display unit 111, an input receiving unit 112, and a display control unit 113. Furthermore, the processor 120 includes an acquisition unit 121, an information processing unit 122, and an output unit 123. In the embodiment, the control system 10 is configured with the acquisition unit 121, the information processing unit 122, and the output unit 123 of the processor 120. Also, in the embodiment, the information provision system 20 is configured with the display unit 111 and the display control unit 113 of the operation panel 110.

The display unit 111 is controlled by the display control unit 113 to display an image or the like. The display unit 111 is, but not limited to, for example, a liquid crystal display, a plasma display, or an organic EL (Electro-Luminescence) display.

The input receiving unit 112 is an input interface that receives input from a user, and is configured with, for example, a keyboard, a touch sensor, a touchpad, or a mouse. The input receiving unit 112 receives a user's input operation and outputs a signal in response to the input operation to the processor 120. Note that, in the present disclosure, the display unit 111 and the input receiving unit 112 are integrally configured with a touchscreen, but they may be configured independently of each other.

The display control unit 113 causes the display unit 111 to display an image or the like based on information output from the processor 120.

The acquisition unit 121 acquires information input by the user at the input receiving unit 112. The acquisition unit 121 also acquires information sent from the server 300 via the communication unit 140 and the network 200. In the embodiment, the acquisition unit 121 acquires health condition information related to the user's health-related preferences, and dish information indicating a dish related to the health condition information. The health condition information and dish information will be described in detail in [Operation], which will be described later.

The information processing unit 122 acquires cooking parameter information, including one or more cooking parameters of a to-be-provided dish provided to the user, based on the health condition information and dish information acquired by the acquisition unit 121. The specific operation of the information processing unit 122 and the cooking parameter information will be described in detail in [Operation], which will be described later.

The output unit 123 outputs a control signal, including the cooking parameter information acquired by the information processing unit 122, to the cooking control unit 150, which controls the cooking device 100.

The storage unit 130 is a recording medium that stores programs executed by the processor 120 and various information used by the control system 10 and the information provision system 20. The recording medium is, for example, a hard disk drive, RAM (Random Access Memory), ROM (Read Only Memory), or semiconductor memory. Note that such a recording medium may be volatile or non-volatile.

The communication unit 140 is a communication interface that communicates with the server 300 via the network 200 through wired or wireless communication. The communication standard used in communication between the communication unit 140 and the server 300 is not particularly limited. Additionally, the communication unit 140 may communicate with the server 300 further via, for example, a relay device such as a router.

The cooking control unit 150 controls the cooking unit 160 in accordance with a control signal output from the output unit 123. For example, the cooking control unit 150 instructs the cooking unit 160 to start cooking or instructs the cooking unit 160 to stop cooking in accordance with the control signal. Additionally, for example, the cooking control unit 150 instructs the cooking unit 160 to change a part or all of the cooking performed by the cooking unit 160 in accordance with the control signal.

The cooking unit 160 performs various types of cooking in accordance with instructions from the cooking control unit 150. The cooking unit 160 is capable of performing cooking according to the type of the cooking device 100. For example, if the cooking device 100 is a pressure cooker type device, the cooking unit 160 can perform cooking by boiling or stewing ingredients.

The server 300 is configured to be capable of communicating with the cooking device 100 via the network 200. The server 300 has a first database 310, a second database 320, and a third database 330.

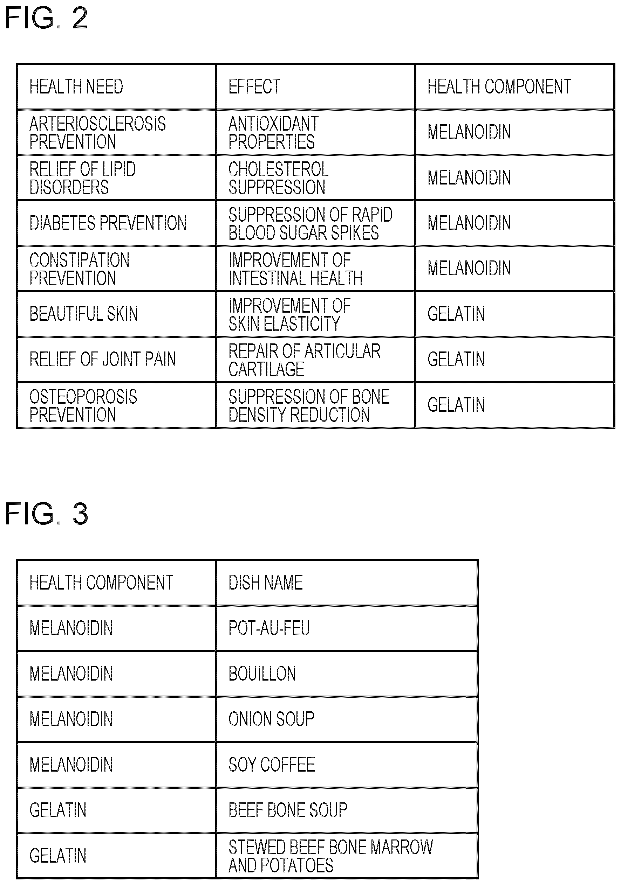

The first database 310 stores first information. The first information refers to information that indicates the correlation between a health need (i.e., the user's health-related preferences), a health component (chemical component) effective in addressing the health need, and the effect of ingesting the health component. Fig. 2 is a diagram illustrating an example of the first information stored in the first database 310. As illustrated in Fig. 2, for example, when the user's health need is "arteriosclerosis prevention", the health ingredient effective for preventing arteriosclerosis is "melanoidin", and the effect of melanoidin is its "antioxidant properties". Additionally, for example, when the user's health need is "beautiful skin", the health component effective for achieving beautiful skin is "gelatin", and the effect of gelatin is "improvement of skin elasticity".

The first information may include combinations of "an effect and a health component" for each health need. For example, (first effect and first health component), ..., (i-th effect and i-th health component) may correspond to arteriosclerosis prevention. Also, (first effect and first health component) may be (antioxidant properties and melanoidin).

The second database 320 stores second information. The second information refers to information indicating the correlation between a health component and a dish. Fig. 3 is a diagram illustrating an example of the second information stored in the second database 320. As illustrated in Fig. 3, for example, when the health component is "melanoidin", the names of dishes in which melanoidin is produced during the cooking process include "pot-au-feu", "bouillon", "onion soup", and "soy coffee". Furthermore, when the health component is "gelatin", the dish names of dishes in which gelatin is produced in the cooking process include "beef bone soup" and "stewed beef bone marrow and potatoes".

Here, melanoidin is produced through the promotion of the Maillard reaction during the cooking process in the cooking device 100, such as a pressure cooker, for example. In addition, gelatin is produced by thermal denaturation of collagen during the cooking process in the cooking device 100, such as a pressure cooker, for example. In other words, collagen is converted to gelatin during the cooking process in the cooking device 100. As such, health components (chemical components) effective in addressing health needs are produced during the cooking process performed by the cooking device 100, i.e., altered (increased or decreased) during the cooking process performed by the cooking device 100.

Note that, although not illustrated in Fig. 3, dishes in which melanoidin is produced during the cooking process include "steak", "roasted coffee", and "miso". In the cooking process of "steak", when a superheated steam type device is used as the cooking device 100, melanoidin is produced through the promotion of the Maillard reaction. In addition, in the cooking process of "roasted coffee", when an IH cooking type device is used as the cooking device 100, melanoidin is produced through the promotion of the Maillard reaction. In addition, in the cooking process of "miso", when a container type device is used as the cooking device 100, melanoidin is produced through the promotion of the Maillard reaction.

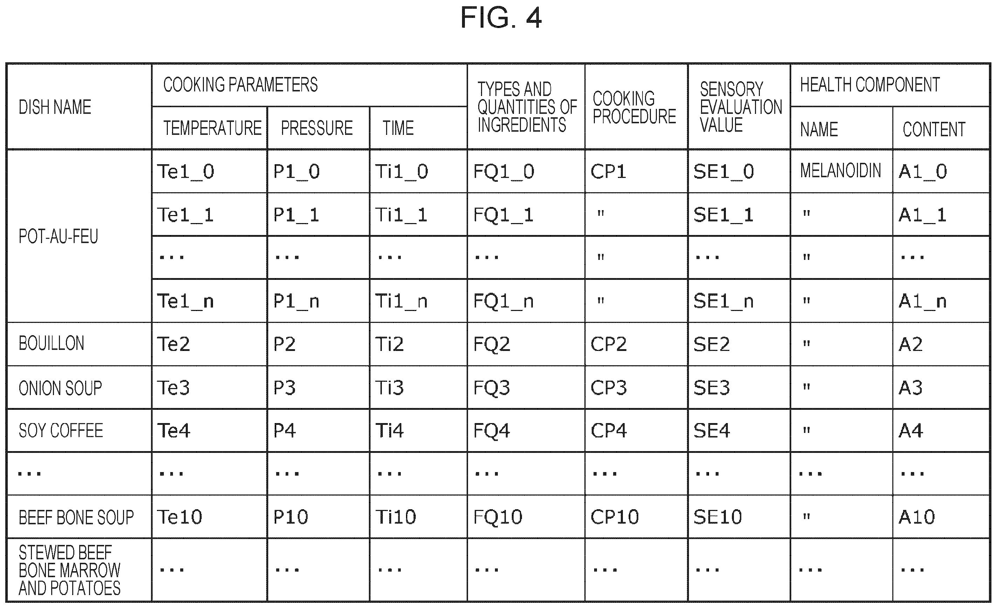

The third database 330 stores third information. The third information refers to information indicating the correlation between a dish (dish name), one or more cooking parameters, a recipe (types and quantities of ingredients, and cooking procedure), the dish's sensory evaluation value (described below), and a health component contained in the dish. Fig. 4 is a diagram illustrating an example of the third information stored in the third database 330. As illustrated in Fig. 4, for example, when the dish name is "pot-au-feu", one or more cooking parameters for producing melanoidin, a health component, in the cooking process include "Te1_0" for the temperature of the cooking unit 160 (cooking device 100), "P1_0" for the pressure of the cooking unit 160, "Ti1_0" for the cooking time, "FQ1_0" for the types and quantities of ingredients, and "CP1" for the cooking procedure. The sensory evaluation value of "pot-au-feu" cooked in this case is "SE1_0", and the melanoidin content is "A1_0".

For example, "Te1_0" refers to 120°C to 160°C, "P1_0" refers to 2 to 15 atm, and "Ti1_0" refers to 30 seconds to 30 minutes. That is, one or more cooking parameters are indicated by ranges of data, as mentioned above. Note that one or more cooking parameters may be represented by a single point of data, such as "Te1_0" at 150°C. Each parameter may be defined either as a range of data or as a single point of data, as mentioned above. For example, when the dish name is "beef bone soup", one or more cooking parameters for producing gelatin, a health component, in the cooking process are "Te10" for the temperature of the cooking unit 160, "P10" for the pressure of the cooking unit 160, "Ti10" for the cooking time, "FQ10" for the types and quantities of ingredients, and "CP10" for the cooking procedure.

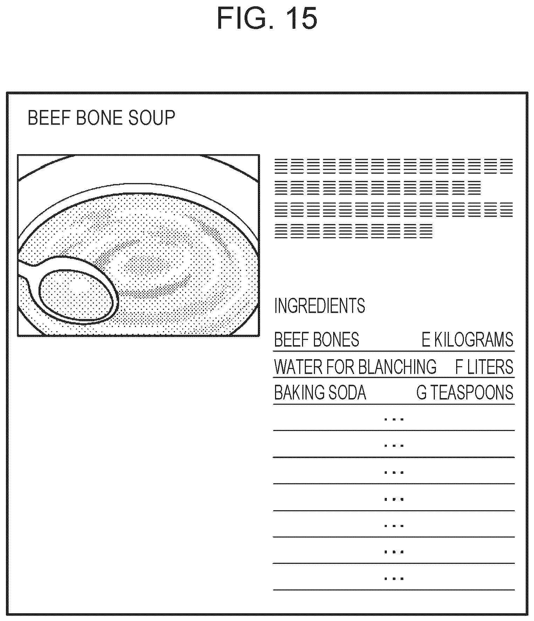

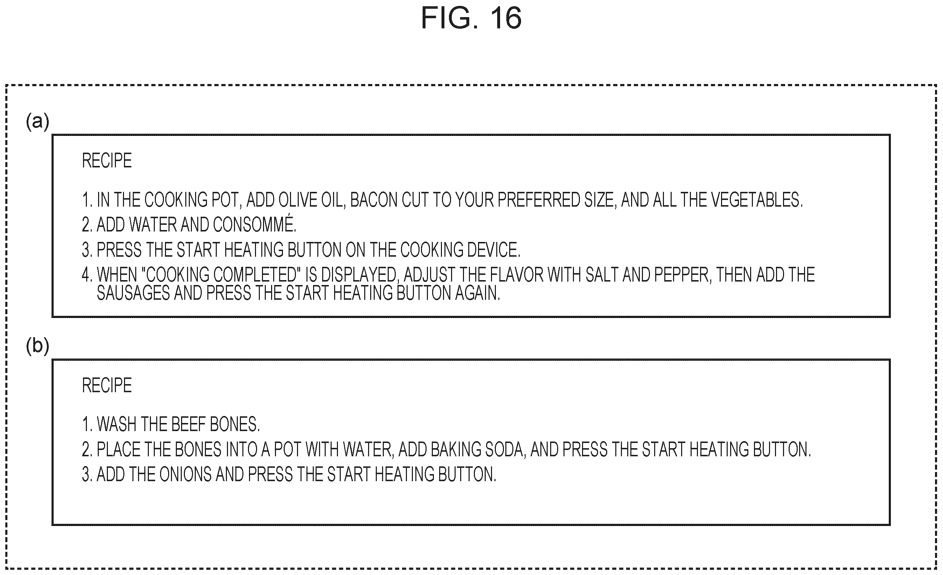

Here, the "FQ... " indicating the types and quantities of ingredients includes parameters such as those indicated in "Ingredients" in Fig. 15, which will be described later. Additionally, the "CP..." indicating the cooking procedure includes steps such as those illustrated in Fig. 16, as described below. The "CP..." indicating the cooking procedure includes the step of processing the ingredients. In other words, the "CP..." indicating the cooking procedure can be said to include the processed states of the ingredients. Here, the processed states of the ingredients include the cut state, size, or the like of the ingredients. For example, the processed states of the ingredients may include the ingredients being cubed, sliced, mashed, or the like. Additionally, for example, the processed states of the ingredients may include the ingredients being cut into 1 cm cubes, sliced to a thickness of 0.5 cm, or the like.

As such, one or more cooking parameters refer to parameters for altering (including producing) a health component (chemical component) corresponding to the user's health-related preferences during the cooking process performed by the cooking device 100.

Additionally, one or more cooking parameters include at least one of a temperature parameter indicating the temperature of the cooking device 100 or a time parameter indicating the cooking time of the cooking device 100. The temperature and time parameters may be used regardless of whether the cooking device 100 is of the pressure cooker type, superheated steam type, IH cooking type, or container type. Furthermore, one or more cooking parameters also include a pressure parameter indicating the pressure of the cooking device 100. The pressure parameter may be used when the cooking device 100 is of the pressure cooker type or superheated steam type.

The quantity of a health component (chemical component) corresponding to the user's health-related preferences may be as follows. This will be described using pot-au-feu included in Fig. 4, for example.

R may be greater than S.

R is the quantity (e.g., A1_0 in Fig. 4) of a chemical component (e.g., melanoidin) contained in a dish (e.g., pot-au-feu) produced based on a predetermined temperature, pressure, time (e.g., Te1_0, P1_0, and Ti1_0 in Fig. 4) and cooking procedure (e.g., CP1 in Fig. 4).

S is the total quantity of the chemical component (e.g., melanoidin) contained in a first quantity of a first ingredient used in the production of the dish (e.g., pot-au-feu), ..., and a p-th quantity of a p-th ingredient. p is one or more integers.

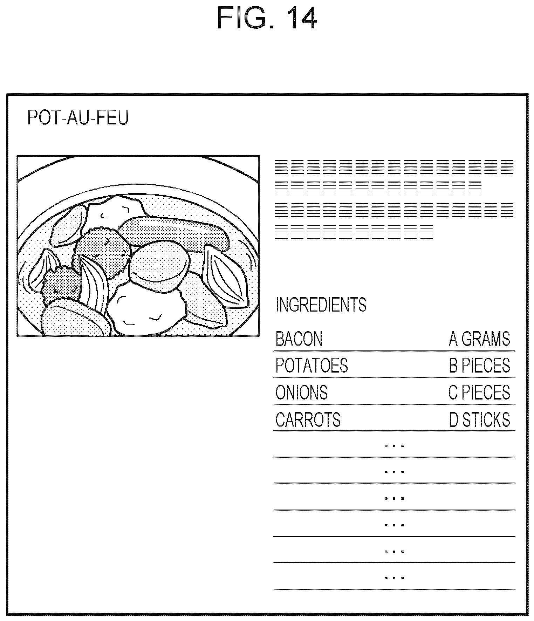

The first quantity of the first ingredient, ..., and the p-th quantity of the p-th ingredient are disclosed, for example, in FQ1_0 in Fig. 4 as well as in FIG. 14.

S may be zero.

In the embodiment, the third information includes one or more reference cooking parameters for each to-be-provided dish provided to the user and one or more modified cooking parameters in which a part or all of the one or more reference cooking parameters have been modified. Here, one or more reference cooking parameters are parameters that can be used to cooking a dish with a standard sensory evaluation value. The sensory evaluation value is determined by the evaluator smelling the aroma of the dish, tasting the dish to check its taste, and so on. The evaluation items for the sensory evaluation values include, for example, aroma (sweet aroma, bitter aroma, and sour aroma), taste (sweetness, bitterness, sourness, umami, and saltiness), and overall evaluation.

For example, when the dish name is "pot-au-feu", one or more cooking parameters mentioned above (the temperature "Te1_0" of the cooking unit 160, the pressure "P1_0" of the cooking unit 160, and the cooking time "Ti1_0") correspond to one or more reference cooking parameters. When "pot-au-feu" is cooked with these reference cooking parameters, the standard sensory evaluation value of "pot-au-feu" is "SE1_0". In the meantime, modified cooking parameters when the dish name is "pot-au-feu" (the temperature "Te1_1" to "Te1_n" of the cooking unit 160 (n is a natural number greater than or equal to 2), the pressure "P1_1" to "P1_n" of the cooking unit 160, and the cooking time "Ti1_1" to "Ti1_n") are parameters that can be used to cook dishes with sensory evaluation values different from the standard sensory evaluation value. If "pot-au-feu" is cooked with these other modified cooking parameters, the sensory evaluation values of "pot-au-feu" are "SE2_1" to "SE2_n".

Note that, regarding the content of a health component (chemical component) contained in the dish, a dish cooked with one or more standard cooking parameters contains a standard quantity of the health component, while a dish cooked with one or more modified cooking parameters contains a different quantity of the health component than the standard quantity.

Note that, in the example illustrated in Fig. 4, the third information for dishes other than "pot-au-feu" includes one or more standard cooking parameters, but may further include one or more modified cooking parameters in which a part or all of the standard cooking parameters have been modified, as described above.

In the embodiment, one or more cooking parameters for each to-be-provided dish in the third information are determined using a trained model generated by prior machine learning. The prior machine learning will be described in detail in [Prior Machine Learning], which will be described later.

### [Operation]

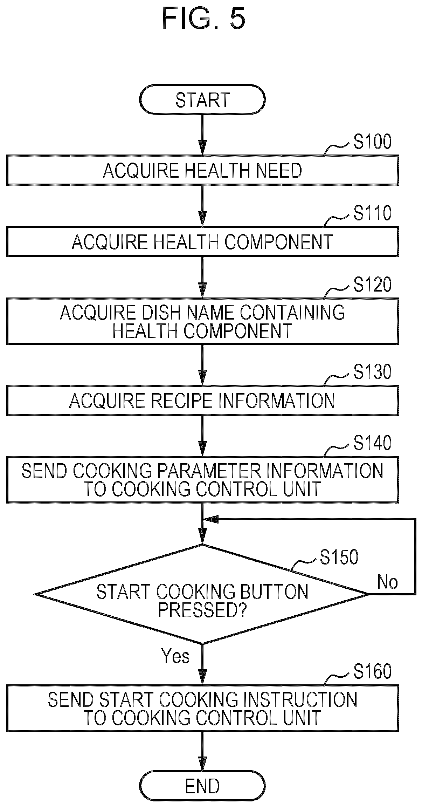

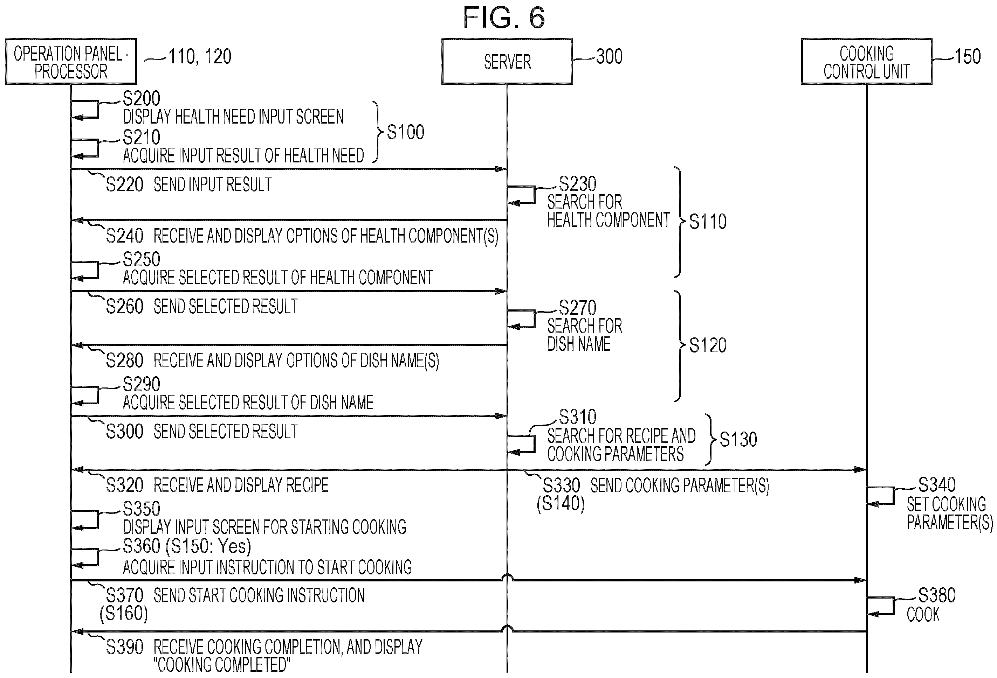

Hereinafter, the operation of the control system 10 (i.e., control method) and the operation of the information provision system 20 (i.e., information provision method) according to the embodiment will be described. Fig. 5 is a flowchart illustrating the overview of the operation of the control system 10 according to the embodiment. Fig. 6 is a sequence diagram illustrating the overview of the operation of each of the control system 10 and the information provision system 20 according to the embodiment.

### (Step S100)

As illustrated in Fig. 5, the acquisition unit 121 of the processor 120 acquires a health need (i.e., the user's health-related preferences). The health need is included in health condition information related to the user's health-related preferences. Step S100 includes step S200 and step S210 illustrated in Fig. 6.

### <Step S200>

First, the display control unit 113 of the operation panel 110 causes the display unit 111 to display a health need input screen. The health need input screen corresponds to a first image that allows input of health condition information related to the user's health-related preferences. The display control unit 113 causes the display unit 111 to display one of the following first images.

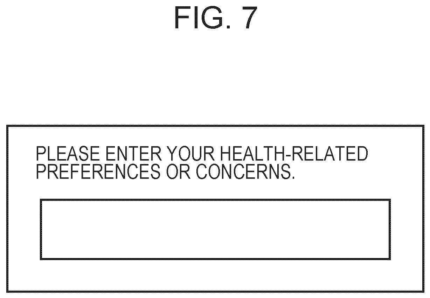

Fig. 7 is a diagram illustrating a first example of the first image displayed on the display unit 111. In the first image illustrated in Fig. 7, a character string prompting the user to enter health-related preferences (including concerns) and a text box allowing the user to enter a character string using a keyboard or similar device are displayed. The user enters one or more words indicating health-related preferences, such as "arteriosclerosis prevention" or "arteriosclerosis", into the text box.

Note that the user may enter a free-form sentence indicating health-related preferences into the text box instead of words. Additionally, the first image may simply display a character string prompting the user to provide audio input of the user's health-related preferences. In this case, the user provides the user's health-related preferences through audio input. In the case where a character string is entered into the text box, the acquisition unit 121 of the processor 120 can identify the user's health-related preferences using appropriate natural language processing techniques. Additionally, in the case where audio input is provided, the acquisition unit 121 of the processor 120 can identify the user's health-related preferences using appropriate speech recognition techniques.

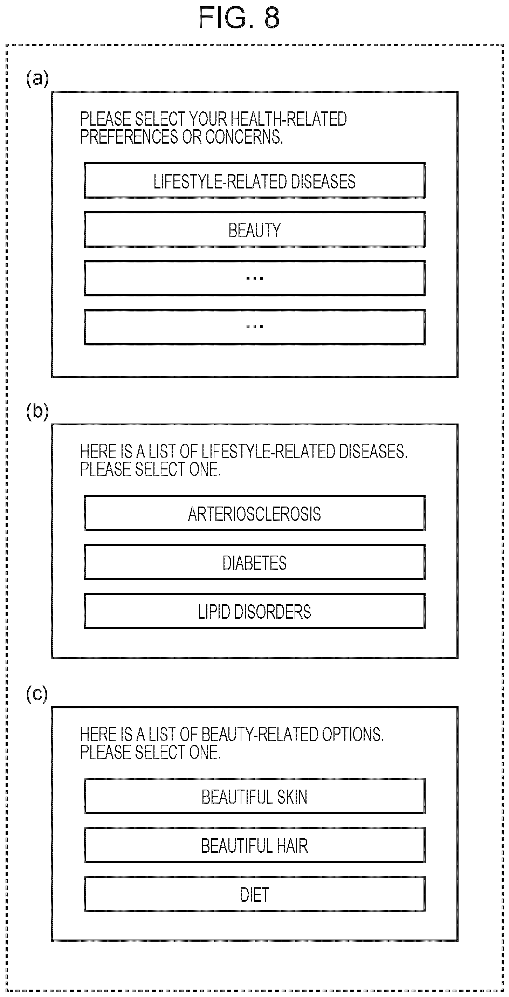

Fig. 8 includes diagrams each illustrating a second example of the first image displayed on the display unit 111. Fig. 8(a) illustrates the first image first displayed on the display unit 111. In the first image illustrated in Fig. 8(a), a character string prompting the user to enter health-related preferences (including concerns), and options, each representing a category of health-related preferences, are displayed. The user selects one category, such as "lifestyle-related diseases" or "beauty", from the categories.

Fig. 8(b) illustrates a first image displayed on the display unit 111 when the category "lifestyle-related diseases" is selected in the first image illustrated in Fig. 8(a). In the first image illustrated in Fig. 8(b), a character string prompting the user to select one option from options, and the options, each representing health-related preferences included in the category of lifestyle-related diseases, are displayed. The user selects one option, such as "arteriosclerosis" or "diabetes", from the options.

Fig. 8(c) illustrates a first image displayed on the display unit 111 when the category "beauty" is selected in the first image illustrated in Fig. 8(a). In the first image illustrated in Fig. 8(c), a character string prompting the user to select one option from options, and the options, each representing health-related preferences included in the category of beauty, are displayed. The user selects one option, such as "beautiful skin" or "beautiful hair", from the options.

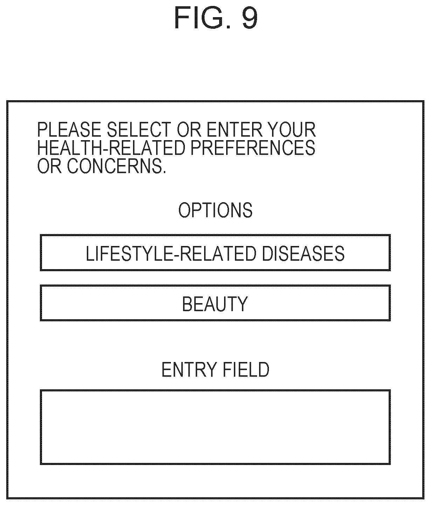

Fig. 9 is a diagram illustrating a third example of the first image displayed on the display unit 111. In the first image illustrated in Fig. 9, a character string prompting the user to enter health-related preferences (including concerns), options, each representing a category of health-related preferences, and a text box allowing the user to enter a character string using a keyboard or similar device are displayed. The user selects one category, such as "lifestyle-related diseases" or "beauty", from the categories. The user additionally enters, for example, a free-form sentence indicating health-related preferences in the selected category into the text box. In this case, as mentioned above, the acquisition unit 121 of the processor 120 can identify the user's health-related preferences using appropriate natural language processing techniques.

### <Step S210>

Referring back to Fig. 6, the user then enters the user's health-related preferences according to the input mode described in step S200, while looking at the first image displayed on the display unit 111 in step S200. As a result, the input receiving unit 112 of the operation panel 110 receives the user's input. Then, the acquisition unit 121 of the processor 120 acquires the input received by the input receiving unit 112, that is, the input result of the health need.

### (Step S110)

Referring back to Fig. 5, the acquisition unit 121 of the processor 120 acquires a health component. The health component is included in health condition information. That is, health condition information includes information indicating a health component (chemical component) corresponding to the user's health-related preferences. Steps S100 and S110 correspond to acquiring health condition information in the control method. Additionally, steps S100 and S110 also correspond to acquiring health condition information in the information provision method. Step S110 includes steps S220 to S250 illustrated in Fig. 6.

### <Step S220>

First, the information processing unit 122 of the processor 120 sends the input result acquired in step S210 to the server 300 via the communication unit 140 and the network 200.

### <Step S230>

Upon receiving the input result sent from the processor 120 via the communication unit 140 and the network 200, the server 300 searches for the health component. Specifically, the server 300 accesses the first database 310 and matches the first information stored in the first database 310 with the received input result, thereby selecting the health component corresponding to the health need included in the input result. For example, if the health need included in the input result is "arteriosclerosis prevention", the server 300 selects "melanoidin" as the health component corresponding to the health need. Note that more than one health component may be selected. If health components correspond to the health need, the server 300 will select these health components.

### <Step S240>

The server 300 sends the search result in step S230 to the processor 120 via the communication unit 140 and the network 200. Upon receiving the search result, the information processing unit 122 of the processor 120 instructs the display control unit 113 to cause the display unit 111 to display one or more health components included in the search result. In response to the instruction, the display control unit 113 of the operation panel 110 causes the display unit 111 to display an image with options of one or more health components. The image corresponds to a first image that receives input of health condition information related to a user's health-related preferences.

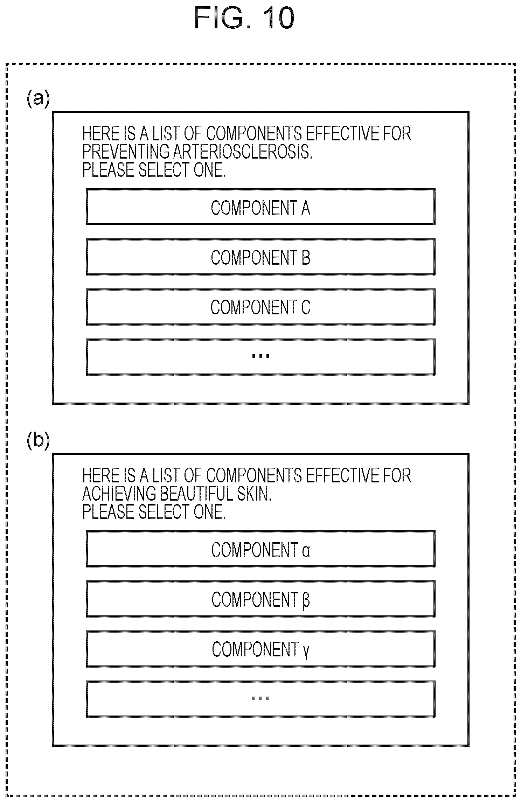

Fig. 10 includes diagrams each illustrating a fourth example of the first image displayed on the display unit 111. Fig. 10(a) illustrates the first image displayed on the display unit 111 when the user selects "arteriosclerosis prevention" as the health need. In the first image illustrated in Fig. 10(a), a character string prompting the user to select one option from options, and the options, each representing a health component effective for preventing arteriosclerosis, are displayed. "Component A" in Fig. 10(a) is, for example, melanoidin. The user selects one option from the options.

Fig. 10(b) illustrates a first image displayed on the display unit 111 when the user selects "beautiful skin" as the health need. In the first image illustrated in Fig. 10(b), a character string prompting the user to select one option from options, and the options, each representing a health component effective for achieving beautiful skin, are displayed. "Component α" in Fig. 10(b) is, for example, gelatin. The user selects one option from the options.

Note that each of the first images displayed in Fig. 10 may include a description of the health component. For example, in the case of the health component "melanoidin", the first image may include a character string explaining that it has antioxidant properties, protects vascular health, and promotes intestinal health.

### <Step S250>

Referring back to Fig. 6, the user then selects one of the health components while looking at the first image displayed on the display unit 111 in step S240. As a result, the input receiving unit 112 of the operation panel 110 receives the user's input. Then, the acquisition unit 121 of the processor 120 acquires the input received by the input receiving unit 112, that is, the selected result of the health component.

### (Step S120)

Referring back to Fig. 5, the acquisition unit 121 of the processor 120 acquires a dish name including the acquired health component. The dish name is included in dish information indicating a dish related to the health condition information. Step S120 corresponds to acquiring dish information in the control method. Step S120 also corresponds to acquiring dish information in the information provision method. Step S120 includes steps S260 to S290 illustrated in Fig. 6.

### <Step S260>

First, the information processing unit 122 of the processor 120 sends the selected result acquired in step S250 to the server 300 via the communication unit 140 and the network 200.

### <Step S270>

Upon receiving the selected result sent from the processor 120 via the communication unit 140 and the network 200, the server 300 searches for the dish name. Specifically, the server 300 accesses the second database 320 and matches the second information stored in the second database 320 with the received selected result, thereby selecting the dish name corresponding to the health component included in the selected result. For example, if the health component included in the selected result is "melanoidin", the server 300 selects "pot-au-feu", "bouillon", "onion soup", and "soy coffee" as dish names corresponding to the health component. Note that more than one dish name may be selected. If dish names correspond to the health component, the server 300 will select these dish names, as mentioned above.

### <Step S280>

The server 300 sends the search result in step S270 to the processor 120 via the communication unit 140 and the network 200. Upon receiving the search result, the information processing unit 122 of the processor 120 instructs the display control unit 113 to cause the display unit 111 to display one or more dish names included in the search result. In response to the instruction, the display control unit 113 of the operation panel 110 causes the display unit 111 to display an image with the one or more dish names. The image corresponds to a second image that receives input of dish information indicating a dish related to the input health condition information.

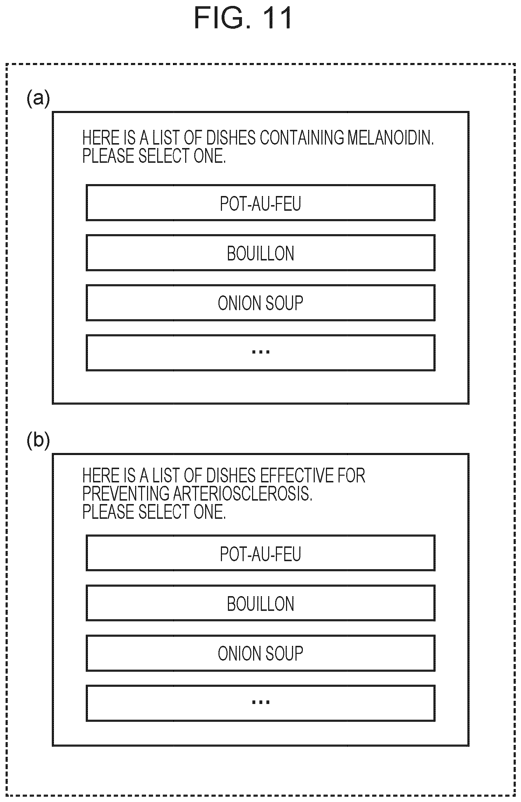

Fig. 11 includes diagrams each illustrating a first example of the second image displayed on the display unit 111. Fig. 11(a) illustrates the second image displayed on the display unit 111 when the user selects "melanoidin" as the health component. In the second image illustrated in Fig. 11(a), a character string prompting the user to select one option from options, and the options, each representing a dish name including melanoidin, are displayed. The user selects one option from the options.

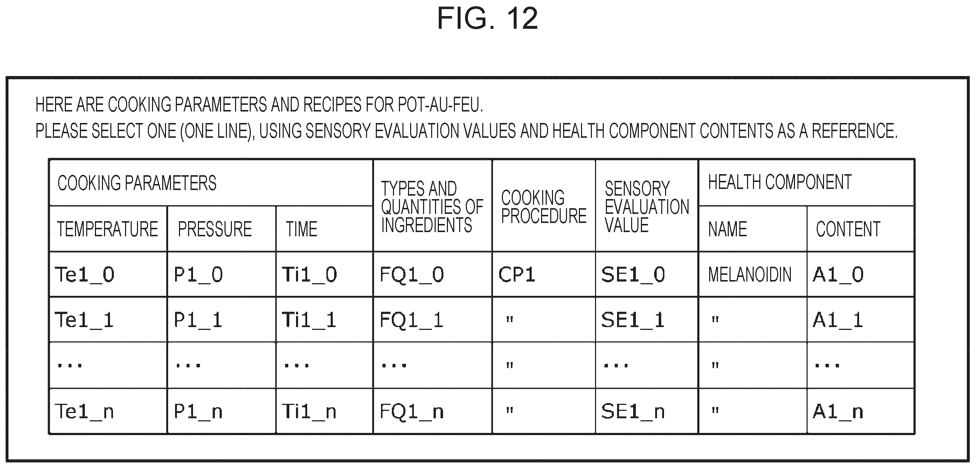

Fig. 12 is a diagram illustrating an example of an image displayed on the display unit 111. Fig. 12 illustrates an image displayed on the display unit 111 when the user selects "pot-au-feu" as the dish name. The image is displayed on the display unit 111 when combinations of one or more cooking parameters and a recipe correspond to the selected dish name. In the image illustrated in Fig. 12, a character string prompting the user to select one option from options, and the options, each representing a combination of one or cooking parameters and a recipe, are displayed. The user selects one option from the options, considering either the dish's sensory evaluation value or the quantity of the health component contained in the dish.

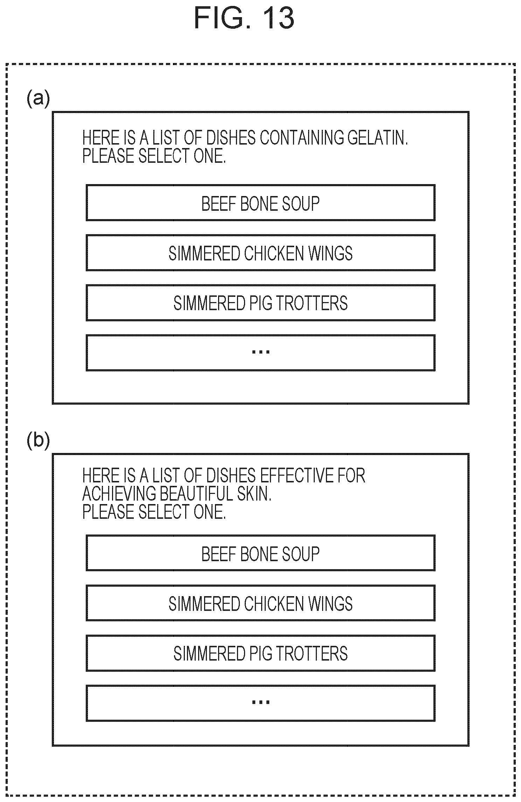

Fig. 13 includes diagrams each illustrating a second example of the second image displayed on the display unit 111. Fig. 13(a) illustrates the second image displayed on the display unit 111 when the user selects "gelatin" as the health component. In the second image illustrated in Fig. 13(a), a character string prompting the user to select one option from options, and the options, each representing a dish name including gelatin, are displayed. The user selects one option from the options.

By the way, step S110 of acquiring a health component may be omitted. Specifically, the server 300 may access both the first database 310 and the second database 320 at the time of receiving the input result in step S230, and may search for the dish name by matching the received input result with both the first information and the second information. That is, the display unit 111 may be caused to display one or more dish names containing a health component corresponding to the health need as a second image, without requiring the user to select a health component.

Fig. 11(b) illustrates a second image displayed on the display unit 111 when "arteriosclerosis prevention" is selected as the health need in step S100. In the second image illustrated in Fig. 11(b), like the second image illustrated in Fig. 11(a), a character string prompting the user to select one option from options, and the options, each representing a dish name containing melanoidin, are displayed. The user selects one option from the options.

Fig. 13(b) illustrates a second image displayed on the display unit 111 when "beautiful skin" is selected as the health need in step S100. In the second image illustrated in Fig. 13(b), like the second image illustrated in Fig. 13(a), a character string prompting the user to select one option from options, and the options, each representing a dish name containing gelatin, are displayed. The user selects one option from the options.

### <Step S290>

Referring back to Fig. 6, the user then selects one dish name while looking at the second image displayed on the display unit 111 in step S280. As a result, the input receiving unit 112 of the operation panel 110 receives the user's input. Then, the acquisition unit 121 of the processor 120 acquires the input, that is, the dish name, received by the input receiving unit 112.

### (Step S130)

Referring back to Fig. 5, the information processing unit 122 of the processor 120 acquires recipe information. Here, the recipe information refers to information related to a dish to be provided, and includes one or more cooking parameters of the to-be-provided dish, the types and quantities of ingredients used in the to-be-provided dish, and the cooking procedure of the to-be-provided dish. That is, the information processing unit 122 of the processor 120 acquires cooking parameter information, including one or more cooking parameters of a to-be-provided dish to be provided to the user, based on the acquired health condition information (here, the health need and the health component) and dish information (here, the dish name). Additionally, the information processing unit 122 of the processor 120 further acquires information indicating at least one of the types, quantities, or processed states of ingredients contained in the to-be-provided dish. Step S130 corresponds to acquiring cooking parameter information in the control method. Step S130 includes steps S300 to S320 illustrated in Fig. 6.

### <Step S300>

First. the information processing unit 122 of the processor 120 sends the selected result acquired in step S290 to the server 300 via the communication unit 140 and the network 200.

### <Step S310>

Upon receiving the selected result sent from the processor 120 via the communication unit 140 and the network 200, the server 300 searches for a recipe and cooking parameters. Specifically, the server 300 accesses the third database 330, and matches the third information stored in the third database 330 with the received selected result, thereby selecting one or more cooking parameters and a recipe (the types and quantities of ingredients, and cooking procedure) corresponding to the dish name included in the selected result. For example, if the dish name included in the selected result is "pot-au-feu", the server 300 selects one or more cooking parameters and a recipe corresponding to the dish name.

Here, if one combination of one or more cooking parameters and a recipe corresponds to the dish name, the server 300 selects this combination. On the other hand, if combinations of one or more cooking parameters and a recipe correspond to the dish name, the server 300 selects a combination of one or more cooking parameters and a recipe that allows the user to cook a dish with the desired sensory evaluation value or a dish containing the desired quantity of the health component. The user can enter the user's desired sensory evaluation value or the user's desired quantity of the health component using the operation panel 110 while looking at, for example, the image displayed on the display unit 111, which is illustrated in Fig. 12.

### <Step S320>

The server 300 sends the search result in step S310, that is, the recipe information, to the processor 120 via the communication unit 140 and the network 200. Upon receiving the recipe information, the information processing unit 122 of the processor 120 instructs the display control unit 113 to cause the display unit 111 to display a recipe included in the recipe information. In response to the instruction, the display control unit 113 of the operation panel 110 causes the display unit 111 to display an image with the recipe. The image corresponds to a third image that represents recipe information related to a to-be-provided dish provided to a user, based on the input health condition information and dish information. Step S320 corresponds to outputting recipe information in the information provision method.

Fig. 14 is a diagram illustrating a first example of the third image displayed on the display unit 111. Fig. 14 illustrates a third image displayed on the display unit 111 when the to-be-provided dish is "pot-au-feu". In the third image illustrated in Fig. 14, an image of "pot-au-feu", a character string indicating a description of "pot-au-feu", and the types and quantities of ingredients used in cooking "pot-au-feu" are displayed.

Fig. 15 is a diagram illustrating a second example of the third image displayed on the display unit 111. Fig. 15 illustrates a third image displayed on the display unit 111 when the to-be-provided dish is "beef bone soup". In the third image illustrated in Fig. 15, an image of "beef bone soup", a character string indicating a description of "beef bone soup", and the types and quantities of ingredients used in cooking "beef bone soup" are displayed.

Fig. 16 includes diagrams each illustrating a third example of the third image displayed on the display unit 111. Fig. 16(a) illustrates the third image displayed on the display unit 111 when the to-be-provided dish is "pot-au-feu". In the third image illustrated in Fig. 16(a), character strings indicating the cooking procedure of "pot-au-feu" are displayed. In the third image illustrated in Fig. 16(b), character strings indicating the cooking procedure of "beef bone soup" are displayed. The user cooks the to-be-provided dish while looking at the third image displayed on the display unit 111.

Here, the first and second steps in the cooking procedure of "pot-au-feu" correspond to a pre-process preceding the cooking process in the cooking device 100; the third step corresponds to a first cooking process; and the fourth step is a post-process following the first cooking process, and it also corresponds to a pre-process preceding a second cooking process, where the user is instructed to "press the 'start heating' button again". Additionally, the first step in the cooking procedure of "beef bone soup" corresponds to a pre-process preceding the cooking process in the cooking device 100, and the second and third steps correspond to the cooking process. As such, the recipe information includes at least one of pre-process information related to a pre-process preceding a cooking process in the cooking device 100, or post-process information related to a post-process following the cooking process in the cooking device 100.

### (Step S140)

Referring back to Fig. 5, either following step S130 or in parallel with S130, the output unit 123 of the processor 120 outputs (sends) a control signal, including one or more cooking parameters included in the recipe information, that is, the acquired cooking parameter information, to the cooking control unit 150, which controls the cooking device 100. Step S140 corresponds to outputting a control signal in the control method. Step S140 corresponds to step S330 illustrated in Fig. 6.

### <Step S340>

Referring back to Fig. 6, upon receiving one or more cooking parameters sent from the processor 120, the cooking control unit 150 sets the one or more cooking parameters.

### <Step S350>

Next, the display control unit 113 of the processor 120 causes the display unit 111 to display an input screen for starting cooking. On the input screen for starting cooking, for example, a start cooking button, which is for starting cooking by the cooking unit 160, is displayed. The user performs an input operation to press the start cooking button after completing, for example, a pre-process preceding the cooking process.

### (Step S150)

Referring back to Fig. 5, the output unit 123 of the processor 120 waits until the user presses the start cooking button (step S150: No). Then, when the user presses the start cooking button, i.e., when the output unit 123 of the processor 120 acquires an input instruction to start cooking (step S150: Yes), the output unit 123 executes step S160. Yes in step S150 corresponds to step S360 illustrated in Fig. 6.

### (Step S160)

The output unit 123 of the processor 120 then outputs (sends) a control signal including a start cooking instruction indicating to start cooking by the cooking unit 160 to the cooking control unit 150. Step S160 corresponds to step S370 illustrated in Fig. 6.

### <Step S380>

As illustrated in Fig. 6, the cooking control unit 150 instructs the cooking unit 160 to start cooking. As a result, the cooking unit 160 performs cooking in accordance with the one or more cooking parameters set by the cooking control unit 150.

### <Step S390>

The cooking control unit 150 instructs the cooking unit 160 to stop cooking when the set cooking time has elapsed. As a result, the cooking unit 160 stops cooking. The cooking control unit 150 then sends a cooking completion signal to the processor 120, indicating that the cooking has been completed. Upon receiving the cooking completion signal, the processor 120 instructs the display control unit 113 to cause the display unit 111 to display the character string "cooking completed" indicating that the cooking has been completed. In response to the instruction, the display control unit 113 of the operation panel 110 causes the display unit 111 to display an image with the character string "cooking completed".

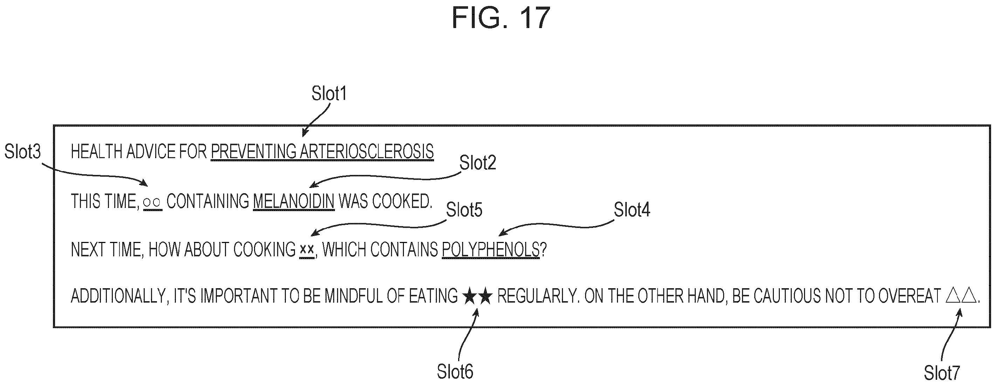

By the way, upon completion of cooking, the display control unit 113 may further cause the display unit 111 to display a fourth image that represents health advice information indicating health advice based on the input health condition information and dish information. Fig. 17 is a diagram illustrating an example of a fourth image displayed on the display unit 111. In the image illustrated in Fig. 17, the character strings excluding "Slot 1" to "Slot 7" are fixed phrases.

In "Slot 1", a character string indicating a health need selected by the user in this instance is displayed. In "Slot 2", a character string indicating a health component selected by the user in this instance, or, if the user has directly selected a dish name, a character string indicating a health component contained in that dish, is displayed. In "Slot 3", a character string indicating a dish name selected by the user in this instance is displayed. In "Slot 4", a character string indicating a health component that corresponds to the health need selected by the user in this instance and that is different from the health component indicated in "Slot 2" is displayed. The health component displayed in "Slot 4" is determined by, for example, referring to the first information stored in the first database 310. In "Slot 5", a character string indicating a dish name containing the health component displayed in "Slot 4" is displayed. The dish name displayed in "Slot 5" is determined by, for example, referring to the second information stored in the second database 320. In "Slot 6", an ingredient name or a dish name that can have a positive effect on the health need selected by the user in this instance is displayed. In "Slot 7", an ingredient name or a dish name that can have a negative effect on the health need selected by the user in this instance is displayed. The ingredient names and dish names displayed in "Slot 6" and "Slot 7" are determined by, for example, referring to general health-related information.

### [Prior Machine Learning]

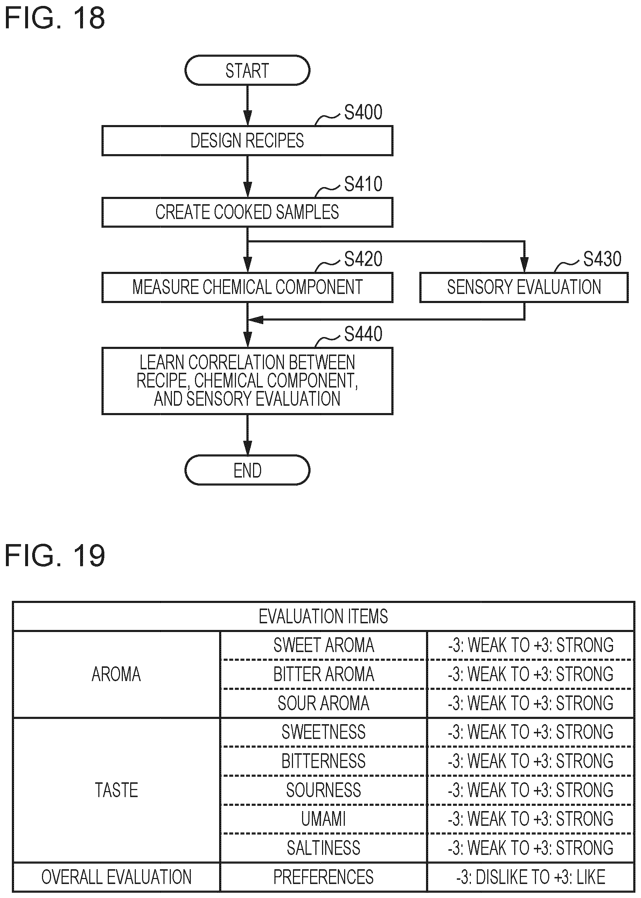

Hereinafter, a method of determining one or more cooking parameters for each to-be-provided dish in the third information stored in the third database 330 using a trained model generated by prior machine learning will be described in detail. Fig. 18 is a flowchart illustrating a process of generating a trained model.

### (Step S400)

First, for each dish to be provided, recipes are designed by varying the recipe parameters (such as one or more cooking parameters, the quantities of the ingredients, and the processed states of the ingredients).

Here, a health component (chemical component) effective for the health need is produced by combining or decomposing the existing component(s) originally contained in the ingredients through chemical reactions. For this reason, the content of a health component contained in a dish depends not only on one or more cooking parameters but also on the quantities and processed states of the ingredients. Therefore, the quantities and processed states of the ingredients are included as the recipe parameters.

By the way, if there are numerous recipe parameters, designing recipes for all possible combinations of these parameters would require an enormous amount of effort in creating cooked samples for the individual recipes, as well as in measuring the chemical components of the cooked samples and performing sensory evaluations, as will be described later. Therefore, in the embodiment, the experimental design method (orthogonal array) is used to reduce the number of recipes to be designed. **In** the experimental design method (orthogonal array), qualitative variables such as the cut states of the ingredients in their processed states are replaced with binary dummy variables.

### (Step S410)

Next, cooked samples are created according to the recipes designed in step S400.

### (Step S420)

Next, for each of the cooked samples created in step S410, the type of the chemical component contained in the cooked sample and the content thereof are measured. For example, if the chemical component is a volatile component, gas chromatography or the like can be used to measure the chemical component. Additionally, for example, if the chemical component is a liquid component, liquid chromatography or the like can be used to measure the chemical component. Furthermore, various analytical methods for nutrient components can be used for measuring chemical components. For example, for proteins, the nitrogen quantification conversion method can be used; and for lipids, the ether extraction method can be used.

### (Step S430)

Next, either following step S420 or in parallel with S420, sensory evaluation (analytical sensory evaluation in the embodiment) is performed for each of the cooked samples created in step S410 by the evaluator smelling the aroma of the cooked sample, tasting the sample to check its taste, and so on. There may be one or more evaluators. If there are evaluators, the average of their evaluation values may be adopted as the sensory evaluation.

Fig. 19 is a diagram illustrating an example of evaluation items for sensory evaluation. As illustrated in Fig. 19, evaluation items for the sensory evaluation values include, for example, aroma (sweet aroma, bitter aroma, and sour aroma), taste (sweetness, bitterness, sourness, umami, and saltiness), and overall evaluation. Here, each evaluation item is rated on a seven-point scale, ranging from "-3" to "+3". For example, if the evaluation item is "sweet aroma", a lower evaluation value indicates a weaker sweet aroma of the cooked sample, while a higher evaluation value indicates a stronger sweet aroma of the cooked sample. For each cooked sample, the evaluator conducts sensory evaluation according to each evaluation item illustrated in Fig. 19.

### (Step S440)

Next, a trained model is generated by learning the correlation between a recipe, its chemical component, and its sensory evaluation. Specifically, the correlation between a recipe and its chemical component, the correlation between the chemical component and its sensory evaluation, and the correlation between the recipe and its sensory evaluation are each modeled using machine learning.

As an example, the concept of modeling the correlation between a recipe and its chemical component will be described. In modeling, first, the measured data of the chemical component will be described. **In** the measured data of the chemical component, the chemical component is the dependent variable, and all the parameters included in the recipe are the independent variables. For the simplicity of the description, it will be described here that the independent variables are the two cooking parameters: the temperature and the pressure of the cooking device 100.

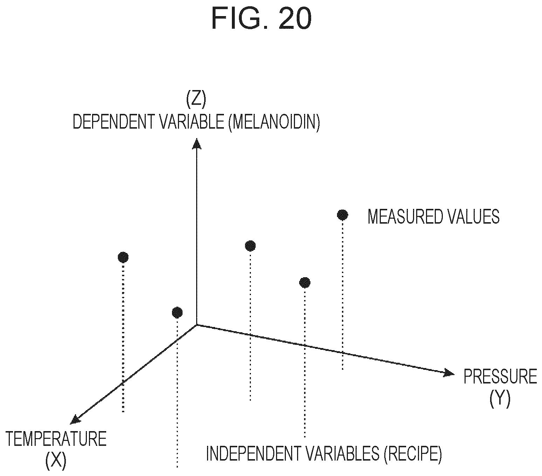

Fig. 20 is a diagram illustrating an example of the measured data of the chemical component. **In** Fig. 20,the X-axis and Y-axis represent the temperature and pressure of the cooking device 100, respectively, which are the independent variables, while the Z-axis represents the chemical component (melanoidin in this case), which is the dependent variable. Then, the values on the dependent variable axis (Z-axis) at the coordinates indicated as black circles in Fig. 20 represent the measured data (measurement values) of the chemical component content, respectively. As illustrated in Fig. 20, changes in the temperature and pressure of the cooking device 100, which are the independent variables, cause the content (produced amount) of the chemical component, which is the dependent variable, to change.

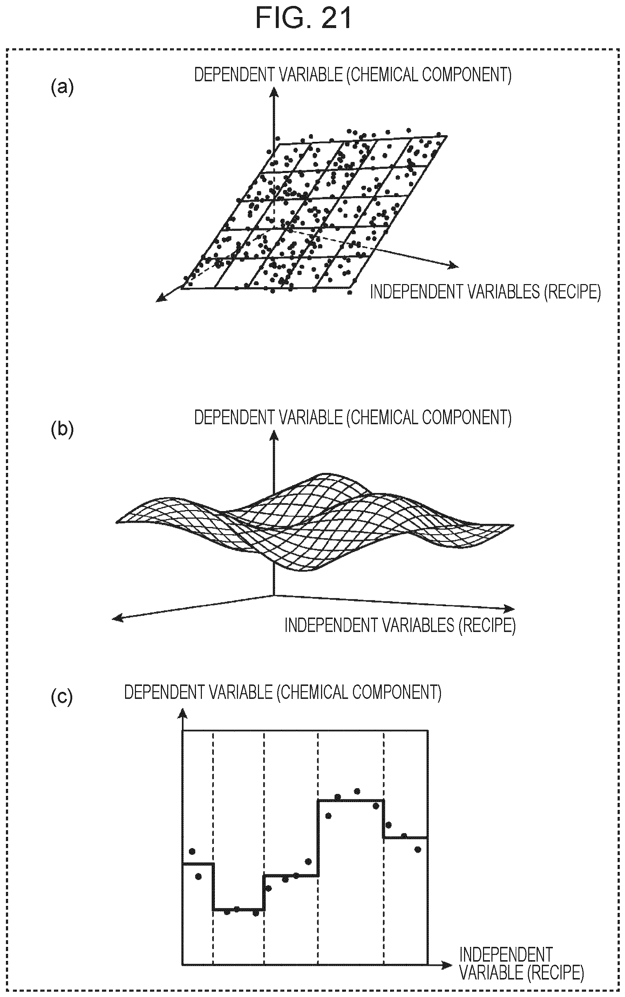

Then, the correlation between the recipe and the chemical component is modeled by performing machine learning on items of measured data. Fig. 21 includes diagrams each illustrating an example of a trained model. For example, if the individual items of measured data can be linearly approximated, machine learning methods such as elastic net regression or support vector regression (linear kernel) can be used to enable linear modeling, as illustrated in Fig. 21(a). **In** Fig. 21(a), the two axes forming the bottom surface represent the independent variables (recipe), while the axis perpendicular to the bottom surface represents the dependent variable (chemical component). For example, if the individual items of measured data cannot be linearly approximated, machine learning methods such as support vector regression (nonlinear kernel) can be used to enable nonlinear modeling, as illustrated in Fig. 21(b). **In** Fig. 21(b), the two axes forming the bottom surface represent the independent variables (recipe), while the axis perpendicular to the bottom surface represents the dependent variable (chemical component). **In** addition, machine learning methods such as random forest regression can be used to enable partitioned linear modeling, as illustrated in Fig. 21(c). **In** Fig. 21(c), the horizontal axis represents the independent variable (recipe), while the vertical axis represents the dependent variable (chemical component).

Subsequently, the above-described machine learning is repeated for each chemical component, with the dependent variable, i.e., the type of chemical component, being changed. As a result, it becomes possible to generate a trained model that is machine-learned to take one or more cooking parameters as input, and to output the content of the chemical component contained in the to-be-provided dish. Using this trained model, it is possible to perform a transformation into a trained model that is machine-learned to take information indicating a to-be-provided dish and its chemical component as input, and to output one or more cooking parameters.

Using the trained model generated in this way, it is possible to calculate the third information stored in the third database 330.

### [Advantages]

As described above, the control system 10 (control method or control program) according to the embodiment can cause a health component (chemical component) related to a user's health-related preferences to change (including being produced) during the cooking process performed by the cooking device 100. Additionally, in the information provision system 20 (information provision method or information provision program) according to the embodiment, it is possible to provide the user with recipe information related to a to-be-provided dish that causes a health component (chemical component) related to the user's health-related preferences to change (including being produced) during the cooking process performed by the cooking device 100. Therefore, in the embodiment, the user can cook a dish containing the aforementioned health component even by using commonly available ingredients, without using rare or special ingredients, for example. As such, the embodiment has the advantage of making it easy to cook a dish containing a chemical component related to the user's health-related preferences.

### (Modifications)

Although the above describes the control system (control method) and the information provision system (information provision method) according to one or more aspects of the present disclosure based on the embodiment, the present disclosure is not limited to the embodiment. As long as it does not depart from the spirit of the present disclosure, various modifications conceived by those skilled in the art, applied to the above embodiment, may also be included in the present disclosure.

**In** the embodiment, a chemical component related to a health need (user's health-related preferences) is a health component, i.e., a component that can have a positive effect on health; however, this is not limited to such components. For example, it is sufficient for the chemical component to include at least one of a component that can have a positive effect on health or a component that can have a negative effect on health. A component that can have a negative effect on health includes, for example, formaldehyde or acetaldehyde. For example, if a chemical component related to a health need is a component that can have a negative effect on health, the cooking parameter information becomes a cooking parameter for reducing that chemical component during the cooking process performed by the cooking device 100.

**In** the embodiment, it is sufficient for the options from which the user selects one in step S250, as illustrated in Fig. 6, to include an option representing a health component (chemical component) produced during the cooking process performed by the cooking device 100. Additionally, the options may further include options representing the existing health components originally contained in the ingredients. **In** this case, it is sufficient for one or more cooking parameters of the to-be-provided dish to be parameters for altering (increasing or decreasing) the existing health components originally contained in the ingredients during the cooking process. For example, if the existing health component is a component that can have a positive effect on the user's health, one or more cooking parameters of the to-be-provided dish may be parameters for increasing the component during the cooking process. Alternatively, for example, if the existing health component is a component that can have a negative effect on the user's health, one or more cooking parameters of the to-be-provided dish may be parameters for reducing the component during the cooking process.

In the embodiment, the server 300 has the first database 310, the second database 320, and the third database 330, but this is not the only possible configuration. For example, the storage unit 130 may have the first database 310, the second database 320, and the third database 330. In this case, the server 300 is unnecessary.

In the embodiment, the information processing unit 122 of the processor 120 accesses the third database 330 to acquire cooking parameter information, but this is not the only possible case. For example, the information processing unit 122 of the processor 120 may acquire cooking parameter information using a trained model generated by prior machine learning. Specifically, the information processing unit 122 of the processor 120 can acquire one or more cooking parameters of the to-be-provided dish by inputting the desired content of a health component (chemical component) into the trained model corresponding to the to-be-provided dish. In this case, the third database 330 is unnecessary.

In the embodiment, for example, when the control system 10 and the information provision system 20 correspond to a terminal (a controller or an information terminal such as a smartphone) that manages cooking devices, one or more cooking parameters may further include the types of the cooking devices.

Note that, in the above-described embodiment, each element may be realized either by dedicated hardware or by executing a software program suitable for each element. Each element may be realized by a program execution unit such as a CPU (Central Processing Unit) or processor, which reads and executes a software program stored on a recording medium such as a hard disk or semiconductor memory.

Note that the following cases are also included in the present disclosure.
(1) At least one of the above-mentioned devices is specifically a computer system composed of a microprocessor, ROM (Read Only Memory), RAM (Random Access Memory), a hard disk unit, a display unit, a keyboard, a mouse, and the like. The RAM or hard disk unit stores a computer program. By the microprocessor operating according to the computer program, at least one of the above-mentioned devices achieves its function. Here, the computer program is composed of a combination of instruction codes that indicate commands to the computer in order to achieve a predetermined function.
(2) A part or all of the elements constituting at least one of the above-mentioned devices may be composed of a single system LSI (Large Scale Integration circuit). The system LSI is ultra-multifunctional LSI in which elements are integrated onto a single chip, and specifically, it is a computer system that includes a microprocessor, ROM, RAM, and the like. The RAM stores a computer program. By the microprocessor operating according to the computer program, the system LSI achieves its function.
(3) A part or all of the elements constituting at least one of the above-mentioned devices may be composed of a detachable IC card or a standalone module for that device. The IC card or module is a computer system composed of a microprocessor, ROM, RAM, and the like. The IC card or module may include the above-mentioned ultra-multifunctional LSI. By the microprocessor operating according to the computer program, the IC card or module achieves its function. The IC card or module may have tamper resistance.
(4) The present disclosure may also be the methods described above. Additionally, the present disclosure may be a computer program that implements these methods on a computer, or it may be a digital signal composed of the computer program.

Additionally, the present disclosure may be a computer program or digital signal recorded on a computer-readable recording medium, such as a flexible disk, hard disk, CD (Compact Disc)-ROM, DVD, DVD-ROM, DVD-RAM, BD (Blu-ray (registered trademark) Disc), semiconductor memory, or the like. Additionally, it may be a digital signal recorded on these recording mediums.

Furthermore, the present disclosure may involve transmitting a computer program or digital signal via telecommunication lines, wireless or wired communication lines, networks such as the Internet, data broadcasting, or the like.

In addition, the program or digital signal may be transferred by recording it onto a recording medium and transporting it, or by transferring the program or digital signal via a network or the like, and it may be implemented by another independent computer system.

### (Conclusion)

As described above, the control system 10 according to a first aspect includes the acquisition unit 121, the information processing unit 122, and the output unit 123. The acquisition unit 121 acquires health condition information, and dish information indicating a dish related to the health condition information. The information processing unit 122 acquires cooking parameter information, including one or more cooking parameters, based on the acquired health condition information and dish information. The output unit 123 outputs a control signal, including the acquired cooking parameter information, to the cooking control unit 150, which controls the cooking device 100. The cooking parameter information includes a cooking parameter for altering a chemical component corresponding to the health condition information during a cooking process performed by the cooking device 100.

Additionally, in the control system 10 according to a second aspect, in the first aspect, the health condition information includes information indicating the chemical component corresponding to the health condition information.

Furthermore, in the control system 10 according to a third aspect, in the second aspect, the one or more cooking parameters include either one or more first cooking parameters corresponding to a dish with a first sensory evaluation value, or one or more second cooking parameters corresponding to a dish with a second sensory evaluation value different from the first sensory evaluation value, and the one or more second cooking parameters are one or more parameters in which a part or all of the one or more first cooking parameters have been modified.

In addition, in the control system 10 according to a fourth aspect, in the first or second aspect, the dish information indicates the dish in which ingredients are combined.

Additionally, in the control system 10 according to a fifth aspect, in any one of the first to third aspects, the information processing unit 122 further acquires information indicating at least one of a type of an ingredient contained in a to-be-provided dish, a quantity of the ingredient, or a processed state of the ingredient.

Furthermore, in the control system 10 according to a sixth aspect, in any one of the first to third aspects, the cooking parameter information includes a cooking parameter for increasing or decreasing a chemical component corresponding to the health condition information during a cooking process performed by the cooking device 100.

In addition, in the control system 10 according to a seventh aspect, in any one of the first to third aspects, the chemical component includes at least one of a component that can have a positive effect on health or a component that can have a negative effect on health.

Additionally, in the control system 10 according to an eighth aspect, in any one of the first to third aspects, the one or more cooking parameters include at least one of a temperature parameter indicating a temperature of the cooking device 100 or a time parameter indicating a cooking time of the cooking device 100.

Furthermore, in the control system 10 according to a ninth aspect, in any one of the first to third aspects, the one or more cooking parameters include a pressure parameter indicating a pressure of the cooking device 100.

In addition, in the control system 10 according to a tenth aspect, in any one of the first to third aspects, the information processing unit 122 acquires the cooking parameter information using a trained model. The trained model is machine-learned to take information indicating a dish and the chemical component as input, and to output the one or more cooking parameters.

Additionally, a control method according to an eleventh aspect is a control method executed by a computer, including: acquiring health condition information (steps S100 and S110); acquiring dish information indicating a dish related to the health condition information (step S120); acquiring cooking parameter information, including one or more cooking parameters, based on the acquired health condition information and dish information (step S130); and outputting a control signal including the acquired cooking parameter information (step S140). The cooking parameter information includes a cooking parameter for altering a chemical component corresponding to the health condition information.

Furthermore, a control program according to a twelfth aspect causes a computer to execute: acquiring health condition information (steps S100 and S110); acquiring dish information indicating a dish related to the health condition information (step S120); acquiring cooking parameter information, including one or more cooking parameters, based on the acquired health condition information and dish information (step S130); and outputting a control signal including the acquired cooking parameter information (step S140). The cooking parameter information includes a cooking parameter for altering a chemical component corresponding to the health condition information.

In addition, the information provision system 20 according to a thirteenth aspect includes the display unit 111 and the display control unit 113. The display control unit 113 causes the display unit 111 to display a first image that receives input of health condition information, to display a second image that receives input of dish information indicating a dish related to the input health condition information, and to display a third image that represents recipe information based on the input health condition information and dish information. The recipe information includes at least one of pre-process information related to a pre-process preceding a cooking process in the cooking device 100, or post-process information related to a post-process following the cooking process in the cooking device 100.

Additionally, in the information provision system 20 according to a fourteenth aspect, in the thirteenth aspect, the display control unit 113 causes the display unit 111 to display a fourth image that represents health advice information indicating health advice based on the input health condition information and dish information.

Furthermore, an information provision method according to a fifteenth aspect is an information provision method executed by a computer, including: acquiring health condition information (steps S100 and S110); acquiring dish information indicating a dish related to the health condition information (step S120); and outputting recipe information based on the acquired health condition information and dish information (step S320). The recipe information includes at least one of pre-process information related to a pre-process preceding a cooking process in the cooking device 100, or post-process information related to a post-process following the cooking process in the cooking device 100.

In addition, an information provision program according to a sixteenth aspect causes a computer to execute: acquiring health condition information (steps S100 and S110); acquiring dish information indicating a dish related to the health condition information (step S120); and outputting recipe information based on the acquired health condition information and dish information (step S320). The recipe information includes at least one of pre-process information related to a pre-process preceding a cooking process in the cooking device 100, or post-process information related to a post-process following the cooking process in the cooking device 100.

### (Miscellaneous)

Modifications of the embodiment of the present disclosure may include, but are not limited to, the following.

### (Item 1)

A method executed by a computer, the method including:
causing a display unit to display a name of a first chemical component, ..., and a name of an n-th chemical component, the name of the first chemical component, ..., and the name of the n-th chemical component corresponding to first information provided by a user, the n being an integer greater than or equal to 1;
causing, by the user, the display unit to display a first dish name, ..., and an m-th dish name corresponding to the i-th chemical component selected from the first chemical component, ..., and the n-th chemical component, the i being an integer greater than or equal to 1, 1 ≤ i ≤ n, the m being an integer greater than or equal to 1;
causing the display unit to display second information including a name and a first quantity of a first ingredient, ..., and a name and a p-th quantity of a p-th ingredient, the first quantity of the first ingredient, ..., and the p-th quantity of the p-th ingredient being used by the user to produce the j-th dish corresponding to the j-th dish name selected from the first dish name, ..., and the m-th dish name, the j being an integer greater than or equal to 1, 1 ≤ j ≤ m, the p being an integer greater than or equal to 1; and
controlling a device storing the first quantity of the first ingredient, ..., and the p-th quantity of the p-th ingredient, based on third information corresponding to the second information, to produce the j-th dish, thereby producing the i-th chemical component while producing the j-th dish;
wherein Ri > Si;
the Ri is a quantity of the i-th chemical component included in the produced j-th dish; and
the Si is a total quantity of a quantity of the i-th chemical component included in the first quantity of the first ingredient, ..., and a quantity of the i-th chemical component included in the p-th quantity of the p-th ingredient.

### (Description of Item 1)

"causing a display unit to display a name of a first chemical component, ..., and a name of an n-th chemical component" is disclosed in, for example, Fig. 10 and its related description.

"the name of the first chemical component, ..., and the name of the n-th chemical component corresponding to first information provided by a user" is disclosed in, for example, Fig. 8 and its related description. The first information may be "arteriosclerosis" or "arteriosclerosis prevention". The name of the first chemical component may be melanoidin.

"causing, by the user, the display unit to display a first dish name, ..., and an m-th dish name corresponding to the i-th chemical component selected from the first chemical component, ..., and the n-th chemical component" is disclosed in, for example, Fig. 11 and its related description.

"causing the display unit to display second information including a name and a first quantity of a first ingredient, ..., and a name and a p-th quantity of a p-th ingredient, the first quantity of the first ingredient, ..., and the p-th quantity of the p-th ingredient being used by the user to produce the j-th dish corresponding to the j-th dish name selected from the first dish name, ..., and the m-th dish name" is disclosed in, for example, Fig. 14 and its related description.

"controlling a device storing the first quantity of the first ingredient, ..., and the p-th quantity of the p-th ingredient, based on third information corresponding to the second information, to produce the j-th dish, thereby producing the i-th chemical component while producing the j-th dish" is disclosed in, for example, Fig. 4 and its related description. The third information may be one or more values corresponding to one or more cooking parameters included in Fig.4. The device may be the cooking device 100.

"Ri > Si; the Ri is a quantity of the i-th chemical component included in the produced j-th dish; and the Si is a total quantity of a quantity of the i-th chemical component included in the first quantity of the first ingredient, ..., and a quantity of the i-th chemical component included in the p-th quantity of the p-th ingredient" is disclosed in, for example, Fig. 4 and its related description.

### (Item 2)

The method according to the item 1,
wherein the Si is 0.

### (Item 3)

The method according to the item 1, wherein:
the first chemical component, ..., and the n-th chemical component, corresponding to the first information, are determined based on a first table;
the first dish name, ..., and the m-th dish name, corresponding to the i-th chemical component, are determined based on a second table;
the second information is determined based on a third table; and
the third information is determined based on the third table.

### (Description of Item 3)

The first table is disclosed in, for example, Fig. 2.

The second table is disclosed in, for example, Fig. 3.

The third table is disclosed in, for example, Fig. 4.

### (Item 4)

The method according to the item 1, wherein:
the device includes the computer; and
the device or a server provided at a location different from a location of the device includes the first table, the second table, and the third table.

### (Description of Item 4)

The server may be the server 300.

### Industrial Applicability

The present disclosure is useful when cooking a dish that can satisfy a user's health-related preferences.

### Reference Signs List

10 control system
20 information provision system
100 cooking device
110 operation panel
111 display unit
112 input receiving unit
113 display control unit
120 processor
121 acquisition unit
122 information processing unit
123 output unit
130 storage unit
140 communication unit
150 cooking control unit
160 cooking unit
200 network
300 server
310 first database
320 second database
330 third database

## Claims

1. A control system comprising:
an acquirer that acquires health condition information and dish information indicating a dish related to the health condition information;
an information processor that acquires cooking parameter information, including one or more cooking parameters, based on the acquired health condition information and dish information; and
an outputter that outputs a control signal, including the acquired cooking parameter information, to a cooking controller that controls a cooking device,
wherein the cooking parameter information includes a cooking parameter for altering a chemical component corresponding to the health condition information during a cooking process performed by the cooking device.

2. The control system according to claim 1, wherein:
the health condition information includes information indicating the chemical component corresponding to the health condition information.

3. The control system according to claim 2, wherein:
the one or more cooking parameters include either one or more first cooking parameters corresponding to a dish with a first sensory evaluation value, or one or more second cooking parameters corresponding to a dish with a second sensory evaluation value different from the first sensory evaluation value; and
the one or more second cooking parameters are one or more parameters in which a part or all of the one or more first cooking parameters have been modified.

4. The control system according to claim 1 or 2, wherein:
the dish information indicates the dish in which ingredients are combined.

5. The control system according to any one of claims 1 to 3, wherein:
the information processor further acquires information indicating at least one of a type of an ingredient, a quantity of the ingredient, or a processed state of the ingredient.

6. The control system according to any one of claims 1 to 3, wherein:
the cooking parameter information includes a cooking parameter for increasing or decreasing a chemical component corresponding to the health condition information during a cooking process performed by the cooking device.

7. The control system according to any one of claims 1 to 3, wherein:
the chemical component includes at least one of a component that can have a positive effect on health or a component that can have a negative effect on health.

8. The control system according to any one of claims 1 to 3, wherein:
the one or more cooking parameters include at least one of a temperature parameter indicating a temperature of the cooking device or a time parameter indicating a cooking time of the cooking device.

9. The control system according to any one of claims 1 to 3, wherein:
the one or more cooking parameters include a pressure parameter indicating a pressure of the cooking device.

10. The control system according to any one of claims 1 to 3, wherein:
the information processor
acquires the cooking parameter information using a trained model, and
the trained model is machine-learned to take information indicating a dish and the chemical component as input, and to output the one or more cooking parameters.

11. A control method executed by a computer, comprising:
acquiring health condition information;
acquiring dish information indicating a dish related to the health condition information;
acquiring cooking parameter information, including one or more cooking parameters, based on the acquired health condition information and dish information; and
outputting a control signal including the acquired cooking parameter information,
wherein the cooking parameter information includes a cooking parameter for altering a chemical component corresponding to the health condition information.

12. A control program causing a computer to execute:
acquiring health condition information;
acquiring dish information indicating a dish related to the health condition information;
acquiring cooking parameter information, including one or more cooking parameters, based on the acquired health condition information and dish information; and
outputting a control signal including the acquired cooking parameter information,
wherein the cooking parameter information includes a cooking parameter for altering a chemical component corresponding to the health condition information.

13. An information provision system comprising:
a display; and
a display controller that causes the display to display a first image that receives input of health condition information,
to display a second image that receives input of dish information indicating a dish related to the input health condition information, and
to display a third image that represents recipe information based on the input health condition information and dish information,
wherein the recipe information includes at least one of pre-process information related to a pre-process preceding a cooking process in a cooking device, or post-process information related to a post-process following the cooking process in the cooking device.

14. The information provision system according to claim 13, wherein:
the display controller further causes the display to display a fourth image that represents health advice information indicating health advice based on the input health condition information and dish information.

15. An information provision method executed by a computer, comprising:
acquiring health condition information;
acquiring dish information indicating a dish related to the health condition information; and
outputting recipe information based on the acquired health condition information and dish information,
wherein the recipe information includes at least one of pre-process information related to a pre-process preceding a cooking process in a cooking device, or post-process information related to a post-process following the cooking process in the cooking device.

16. An information provision program causing a computer to execute:
acquiring health condition information;
acquiring dish information indicating a dish related to the health condition information; and
outputting recipe information based on the acquired health condition information and dish information,
wherein the recipe information includes at least one of pre-process information related to a pre-process preceding a cooking process in a cooking device, or post-process information related to a post-process following the cooking process in the cooking device.
